# EUROPEAN PATENT SPECIFICATION

(11) **EP 3 919 917 B1**
(45) Date of publication and mention of the grant of the patent: **17.09.2025**
(21) Application number: 20748065.8
(22) Date of filing: 29.01.2020
(51) Int. Cl.: G01N 37/00, C12M 1/00, C12M 1/26, C12N 15/10, C12N 15/113, C12Q 1/6806, G01N 1/40, B82Y 5/00, B82Y 15/00, B82Y 30/00, B01L 3/00

(54) **BIOMOLECULE RECOVERING DEVICE AND METHOD, AND BIOMOLECULE ANALYZING DEVICE AND METHOD**
VORRICHTUNG UND VERFAHREN ZUR RÜCKGEWINNUNG VON BIOMOLEKÜLEN SOWIE VORRICHTUNG UND VERFAHREN ZUR ANALYSE VON BIOMOLEKÜLEN
DISPOSITIF ET PROCÉDÉ DE RÉCUPÉRATION DE BIOMOLÉCULES, ET DISPOSITIF ET PROCÉDÉ D'ANALYSE DE BIOMOLÉCULES

(30) Priority: 30.01.2019 JP 2019014906
(43) Date of publication of application: 08.12.2021
(73) Proprietor: Craif Inc., Tokyo 1130034 (JP)
(72) Inventor: ONOSE, Ryuichi, Tokyo 1130033 (JP); AKATSU, Takeshi, Tokyo 1130033 (JP)
(74) Representative: Michalski Hüttermann & Partner Patentanwälte mbB
(86) International application number: PCT/JP2020/003284
(87) International publication number: WO 2020/158832

(56) References cited:
- WO-A1-2006/122697
- WO-A1-2015/137427
- WO-A1-2015/137427
- WO-A1-2017/154614
- CN-A- 107 085 107
- JP-A- 2017 203 763
- JP-B2- 5 171 812
- US-A1- 2011 108 424
- US-A1- 2013 225 416
- US-A1- 2014 034 132
- US-A1- 2014 083 859
- US-B1- 7 290 667
- TAKAO YASUI ET AL: "Unveiling massive numbers of cancer-related urinary-microRNA candidates via nanowires", SCIENCE ADVANCES, vol. 3, no. 12, 15 December 2017 (2017-12-15), pages e1701133, XP055705304, DOI: 10.1126/sciadv.1701133
- YI-TSUNG LU ET AL: "NanoVelcro Chip for CTC enumeration in prostate cancer patients", METHODS, vol. 64, no. 2, 1 December 2013 (2013-12-01), NL, pages 144 - 152, XP055231535, ISSN: 1046-2023, DOI: 10.1016/j.ymeth.2013.06.019

## Description

### TECHNICAL FIELD

The present disclosure relates to collection of biomolecules.

### BACKGROUND ART

Various biomolecules can be used as an indicator to represent a physiological state in a living body (e.g., a biomarker). In order to separate, extract, and collect biomolecules from solutions such as body fluids, there are physical methods such as centrifugation and filters, and chemical methods such as aggregation methods by reagents.
US 2011/108424 A1 refers to a device for separating biomolecules from a fluid comprises a microfluidic component provided with at least one microchannel having at least one of the walls supporting a plurality of nanotubes or nanowires. The component comprises at least one electrode electrically connected to at least a part of the nanotubes or nanowires and the device comprises means for applying a voltage between the electrode and the fluid. The nanotubes or nanowires are divided into several active areas in which the nanotubes or nanowires have a different density.
US 7 290 667 B1 refers to a microfluidic sieve having a substrate with a microfluidic channel, and a carbon nanotube mesh. The carbon nanotube mesh is formed from a plurality of intertwined free-standing carbon nanotubes which are fixedly attached within the channel for separating, concentrating, and/or filtering molecules flowed through the channel. In one embodiment, the microfluidic sieve is fabricated by providing a substrate having a microfluidic channel and growing the intertwined free-standing carbon nanotubes from within the channel to produce the carbon nanotube mesh attached within the channel.
WO 2006/122697 A1 refers to a microfluidic component comprises at least one channel delineated by a top wall and a bottom wall and two opposite side walls. The distance between the top wall and the bottom wall of the channel is greater than or equal to 25 micrometers and first and second sets of nanotubes are respectively borne by the two opposite side walls for the component to present a particularly high ratio between the contact surface and the available volume and a limited overall surface size.
US 2014/034132 A1 refers to a microfluidic device having superhydrophilic bi-porous interfaces are provided, along with their methods of formation. The device can include a substrate defining a microchannel formed between a pair of side walls and a bottom surface and a plurality of nanowires extending from each of the side walls and the bottom surface.
US 2014/083859 A1 refers to a method for producing, in a substrate, an enclosed channel or enclosed cavity comprising at least one functional nanofiber, the method comprising the steps of providing a first substrate and a second substrate; forming a channel or cavity on the first substrate or the second substrate; electrospinning at least one functional nanofiber on the first substrate; assembling the first and second substrates, wherein the first substrate is placed over the second substrate, or the second substrate is placed over the first substrate; and bonding the first substrate and the second substrate to form the substrate, thereby forming an enclosed channel or enclosed cavity comprising the at least one functional nanofiber in the substrate.
WO 2015/137427 A1 refers to a biomolecule extraction chip in which nanowires inside a microchannel do not readily detach, even if the inflow velocity of a sample is increased. By providing the biomolecule extraction chip which includes a substrate, the microchannel formed on the substrate, and the nanowires formed on the microchannel, and in which a portion of the nanowires is embedded in the microchannel, the nanowires inside the microchannel do not readily detach.
Takao Yasui ET AL: "Unveiling massive numbers of cancer-related urinary-microRNA candidates via nanowires", Science Advances, vol. 3, no. 12, 1 Oecember 2017 (2017-12-01), page e1701133.
Yi-Tsung Lu ET AL: "NanoVelcro Chip for CTC enumeration in prostate cancer patients", Methods, vol. 64, no. 2, 1 Oecember 2013 (2013-12-01 ), pages 144-152.

However, existing methods may not detect them in small sample volumes or at low concentrations. In addition, those methods are sometimes not practical, for example, they are costly.

### SUMMARY OF INVENTION

The present disclosure includes a device of collecting biomolecules, comprising:
a fluid chamber that is at least partially rectangular parallelepiped;
nanowires disposed on both of opposing inner walls of at least one pair of the rectangular parallelepiped walls of the fluid chamber, wherein the fluid chamber comprises:
   a pair of substrates having a substantially flat surface and having nanowires disposed on the substantially flat surface, the pair of substrates being bonded so that the surfaces on which the nanowires are disposed oppose each other; and
   a spacer sandwiched between the pair of substrates; the spacer configured to define a space having the nanowires between the opposing surfaces of the pair of substrates. The present disclosure also includes a method of collecting biomolecules.

### BRIEF DESCRIPTION OF DRAWINGS

FIG. 1 illustrates a cross-sectional view schematically showing a fluid device according to an example not part of the invention.
FIG. 2 illustrates a cross-sectional view schematically showing a fluid device according to an example not part of the invention.
FIG. 3 illustrates a cross-sectional view schematically showing a fluid device according to an embodiment.
FIG. 4 illustrates a cross-sectional view schematically showing a fluid device according to an example not part of the invention.
FIG. 5 illustrates a cross-sectional view schematically showing a fluid device according to an example not part of the invention.
FIG. 6 illustrates a cross-sectional view schematically showing a fluid device according to an embodiment.
FIG. 7 illustrates a cross-sectional view schematically showing a fluid device according to an embodiment.
FIG. 8 illustrates a cross-sectional view schematically showing a fluid device according to an example not part of the invention.
FIG. 9 illustrates a cross-sectional view schematically showing a fluid device according to an example not part of the invention.
FIG. 10 illustrates a cross-sectional view schematically showing a fluid device according to an example not part of the invention.
FIG. 11 illustrates a cross-sectional view schematically showing a fluid device according to an example not part of the invention.
FIG. 12 illustrates a top view schematically showing an inner wall of a fluid device according to an example not part of the invention.
FIG. 13 illustrates a top view schematically showing an inner wall of a fluid device according to an example not part of the invention.
FIG. 14 illustrates a top view schematically showing an inner wall of a fluid device according to an example not part of the invention.
FIG. 15 illustrates a top view schematically showing an inner wall of a fluid device according to an example not part of the invention.
FIG. 16 illustrates a top view schematically showing an inner wall of a fluid device according to an example not part of the invention.
FIG. 17 illustrates a top view schematically showing an inner wall of a fluid device according to an example not part of the invention.
FIG. 18 illustrates a top view schematically showing an inner wall of a fluid device according to an example not part of the invention.
FIG. 19 illustrates a top view schematically showing an inner wall of a fluid device according to an example not part of the invention.

### DESCRIPTION OF EMBODIMENTS

A biomolecule may be a biological substance. A biological substance is a generic term for an organic compound of a polymer contained in a living body and functioning with respect to life phenomena, and refers to, for example, a protein, a lipid, a nucleic acid, a hormone, a sugar, an amino acid, or the like. The biomolecule may be a complex of a biomolecule, for example, a complex of a protein, and may be a multiprotein complex. The biomolecule may be a nucleic acid. The biomolecule may be a vesicle. The substance to be collected (extracted, accumulated, or the like. Hereinafter, it is also referred to as "collection".) may not be a biomolecule or may be a non-biomolecule. The substance to be collected may be an inorganic molecule, an organic molecule, or the like.

The biomolecule may be a ribonucleic acid (RNA) or may comprise a ribonucleic acid (RNA). The RNA may be, but is not limited to, messenger RNA (mRNA), transfer RNA (tRNA), ribosomal RNA (rRNA), non-coding RNA (ncRNA), microRNA (miRNA), ribozymes, double-stranded RNA (dsRNA), or the like, and may include a plurality thereof. The RNA may be modified. RNA or miRNA may be involved in the development or progression of a cancer, a cardiovascular disease, a neurodegenerative disease, a psychiatric disease, a chronic inflammatory disease, or the like. miRNA may be a type of RNA that promotes or positively regulates canceration (onco miRNA (oncogenic miRNA, cancer-promoting miRNA)) or a type of RNA that suppresses or negatively regulates canceration (Tumor Suppressor miRNA (cancer-suppressing miRNA)). The biomolecule may be an exosome, an exosome complex.

The nucleic acid may be a deoxyribonucleic acid (DNA) or may comprise DNA.

The biomolecule may be an organelle or a vesicle. The vesicle may be, but is not limited to, a vacuole, a lysosome, a transport vesicle, a secretion, gas vesicle, an extracellular matrix vesicle, an extracellular vesicle, or the like, or may include a plurality thereof. The extracellular vesicle may be, but is not limited to, an exosome, an exotome, a shedding microvesicle, a microvesicle, a membrane particle, a plasma membrane, a poptotic vesicle, or the like. The vesicle may contain nucleic acids.

The biomolecule may be, but is not limited to, a cell and may include a cell. The cell may be a red blood cell, a white blood cell, an immune cell, or the like. The biomolecule may be a virus, a bacterium, or the like.

The solution may be a body fluid, a liquid derived from a body fluid (a diluent, a treatment liquid, or the like). The solution may be a solution that is not a body fluid (derived from a non-body fluid), may be an artificially prepared liquid, or may be a mixture of a solution derived from a body fluid or a body fluid and a solution derived from a non-body fluid. The solution may be a solution to be used for sample measurements and may be a solution to be used for measurements for calibration. The solution may be used as a stock solution, or may be a liquid in which the stock solution is diluted or concentrated. The solution may be a standard solution or a calibration solution. The sample to be measured may be a specimen. The solution may contain a physiological buffer such as phosphate buffered saline (PBS) or N-tris(hydroxymethyl)methyl-2-aminoethanesulfonic acid buffer (TES), including the substance to be collected. The body fluids may include an additive. For example, a stabilizing agent or a pH adjusting agent may be added to the additive.

The "body fluid" may be a solution. The body fluid may be in a liquid state or may be in a solid state, for example, a frozen state. The solution may include a substance to be collected, such as a biomolecule, or may not include a substance to be collected, and may include a substance for measuring a substance to be collected.

The body fluid may be a body fluid of an animal. The animal may be a reptile, a mammal, an amphibian. The mammal may be a dog, cat, cow, horse, sheep, pig, hamster, mouse, squirrel, or a primate such as monkey, gorilla, chimpanzee, bonovo, human and the like.

The body fluid may be lymph fluid, tissue fluid such as interstitial fluid, intercellular fluid, interstitial fluid, and the like, or may be body cavity fluid, serosal fluid, pleural fluid, ascites fluid, capsular fluid, cerebrospinal fluid, joint fluid (synovial fluid), and aqueous humor of the eye (aqueous). The body fluid may be a digestive fluid such as saliva, gastric juice, bile, pancreatic juice, intestinal fluid, and the like, or may be sweat, tears, nasal mucus, urine, semen, vaginal fluid, amniotic fluid, milk, or the like.

"Urine" means liquid excreta produced by the kidneys. Urine may be a liquid or substance that has been excreted outward via the urethra or may be a liquid or substance that has been accumulated in the bladder. "Saliva" means a secretion that is secreted into the oral cavity from the salivary glands.

The body fluid may be extracted or accumulated / collected from the body using an extractor such as a syringe. The solution may be a body fluid of a healthy subject, may be a body fluid of a subject with a particular disease, for example but not limited to, lung cancer, liver cancer, pancreatic cancer, bladder cancer, and prostate cancer, or may be a body fluid of a subject suspected of suffering from a particular disease.

The extraction may be adsorption. The substance to be measured may be captured in a device or a fluid chamber or adsorbed on a portion of the interior thereof.

A part or all of the members constituting the fluid chamber or the fluid channel such as a substrate or a spacer may be formed of an inorganic material, or may be formed of an organic material. The inorganic material forming the substrate may be, for example, a metal, silicon, or another semiconductor material, or an insulating material such as glass, ceramics, or a metal oxide.

A fluid chamber or a channel such as a substrate or a spacer may be formed of a polymer material. The polymeric material may be a natural resin, may be a synthetic resin, or may be a mixture thereof. The synthetic resin may be a thermosetting resin, may be a thermoplastic resin, or may be another resin.

The thermosetting resin may be, for example but not limited to, phenol resin (PF), epoxy resin (EP), melamine resin (MF), urea resin (urea resin, UF), unsaturated polyester resin (UP), alkyd resin, polyurethane (PUR), thermosetting polyimide (PI), or the like.

The thermoplastic resin may be, for example but not limited to, a general purpose plastic such as polyethylene (PE), high density polyethylene (HDPE), middle density polyethylene (MDPE), low density polyethylene (LDPE), polypropylene (PP), polyvinyl chloride (PVC), polyvinylidene chloride, polystyrene (PS), polyvinyl acetate (PVAc), polyurethane (PUR), Teflon-(polytetrafluoroethylene, PTFE), ABS resin (acrylonitrile butadiene styrene resin), AS resin, acrylic resin (PMMA), or the like; may be an engineering plastic such as polyamide (PA), nylon, polyacetal (POM), polycarbonate (PC), modified polyphenylene ether (m-PPE, modified PPO), polyethylene terephthalate (PET), glass fiber reinforced polyethylene terephthalate (GF minus PET), polybutylene terephthalate (PBT), cyclic polyolefin (COP) or the like; or may be a superengineering plastic such as polyphenylene sulfide (PPS), polytetrafluoroethylene (PTFE) (commonly referred to as Teflon (registered trademark)), polysulfone (PSF), polyether sulfone (PES), amorphous polyarylate (PAR), liquid crystal polymer (LCP), polyether ether ketone (PEEK), thermoplastic polyimide (PI), polyamideimide (PAI), or the like.

A part or all of the members constituting a flow channel chamber may be flat, may have a curved surface, or may have another shape (for example, bent, or the like).

In some embodiments, a flow channel chamber or flow channel (also referred to simply as a flow channel chamber in the present disclosure) may have a plurality of inner walls. The flow channel chamber or flow channel may have a space substantially surrounded by a plurality of inner walls. The flow channel chamber or flow channel may have a polygonal cross-section in part. The polygon may be, for example, triangle, rectangle, pentagon, hexagon, octagon, or the like. The plurality of inner walls may be composed of a flat inner wall, an inner wall having a curved surface, a combination thereof.

In some embodiments, the flow channel chamber or flow channel may have a member forming an inner wall inside, in addition to the inner walls forming the internal space. For example, a wall or a columnar structure may be provided in the flow channel chamber. The surfaces may constitute an inner wall. The wall or columnar structure may have a structure that protrudes or recesses from the inner wall, and may have a structure that partially traverses the internal space continuously from one inner wall to the opposing inner wall or to another inner wall.

In some embodiments, the flow channel chamber or flow channel may have a curved and continuous inner wall. For example, the flow channel chamber or the flow channel may have a shape in which a cross section of a part thereof is configured by a circle, an ellipse, or other curves.

In some embodiments, the flow channel chamber may constitute a closed space surrounded by an inner wall. The solution may be introduced through an openable and closable inlet. In some embodiments, the flow channel chamber may have an inlet and an outlet for the solution. In some embodiments, the flow channel chamber is configured as a flow channel and may be in fluid communication with other chambers or components. In some embodiments, the fluid chamber may have an air hole.

Nanowires may be disposed substantially perpendicular to the wall surface on which they are disposed. Nanowires may be disposed non-perpendicular to the wall surface where they are disposed. A plurality of nanowires may be disposed at different angles to the wall surface where they are disposed. Nanowires may be disposed parallel to the wall surface where they are disposed. The nanowires may have branched chains. The nanowires may have a single structure without branched chains or unbranched. The plurality of nanowires may include nanowires having branched chains and unbranched nanowires. The nanowires may be periodically disposed at regular intervals on the wall surface where they are disposed. The nanowires may be disposed randomly or aperiodically on the wall surface where they are placed. The nanowires may be formed by growing from a starting point on the wall surface. The nanowires may be disposed to extend from a starting point on the wall surface.

In some embodiments, the nanowires may be directly fixed to the material forming the flow channel or fluid chamber. The nanowires may be grown directly from the wall surface.

In some embodiments, the nanowires may be partially embedded in the wall surface. The nanowires may be grown starting from a growth wire embedded in the wall surface.

In some embodiments, the nanowires may be disposed throughout the wall surface. In some embodiments, the nanowires may be disposed on a part of the wall surface.

The nanowires may not be physicochemically fixed to the inner wall. For example, the nanowires or assemblies thereof may be disposed in contact with or near the inner wall. The nanowires may be macroscopically immobilized or moved by the introduction of solution. In some embodiments, the nanowires may be mechanically in contact with the inner wall, mechanically in substantial contact with the inner wall, or mechanically substantially fixed in proximity to the inner wall. For example, an aggregate of nanowires (for example, macroscopically or microscopically sheet-like aggregate) may be fixed to the inner wall using an insert, an adhesive, or the like.

In some embodiments, a surface treatment such as an activation treatment, a hydrophilic treatment, a heat treatment, a hydrothermal treatment and the like may be performed on the surface of the substrate (inner wall) or the surface of the catalyst layer on which the nanowires are formed or grown. The surface treatment may be, for example, a plasma treatment, particle (ion, radical, neutral atom, or the like) beam irradiation, light (electromagnetic wave) irradiation such as UV, EUV and the like, electron beam irradiation, mechanical treatment such as polishing, or the like. The surface treatment may be, for example, a treatment for increasing the presence of oxygen which is bonded to a metal to become Lewis acid.

As used herein, "nanowire" means a rod-like, wire-like structure having a size such as a cross-sectional shape or a diameter on the order of nanometers (for example but not limited to, a diameter of 1 to hundreds of nanometers).

The material of the nanowires may be an inorganic material or an organic material. The nanowires may be or include a metal, a non-metal, a semiconductor, a mixture or alloy thereof, or an oxide or a nitride thereof. The material of the nanowire may be or include a polymeric material. The nanowires may be wires, whiskers, fibers, and mixtures or composites thereof.

Metals used in the material of the nanowires are for example but not limited to, typical elements (alkali metal: Li, Na, K, Rb, Cs, alkaline earth metal: Ca, Sr, Ba, Ra), magnesium group elements: Be, Mg, Zn, Cd, Hg, aluminum group elements: Al, Ga, In, rare earth elements: Y, La, Ce, Pr, Nd, Sm, Eu, tin group elements: Ti, Zr, Sn, Hf, Pb, Th, iron group elements: Fe, Co, Ni, earth acid elements: V, Nb, Ta, chromium group elements: Cr, Mo, W, U, manganese group elements: Mn, Re, noble metals (copper group, coin metal): Cu, Ag, Au, platinum group elements: Ru, Rh, Pd, Os, Ir, Pt, natural radioactive elements: U and Th as a mother radioactive disintegration products: U, Th, Ra, Rn,
actinoid, transuranic elements: Np, Pu, Am, Cm, Bk, Cf, Es, Fm, Md, No, and the like, uranium or later elements, or an alloy thereof. The nanowires may be an oxide of any one of the above metals or alloys, or alloys or mixtures thereof, and may include an oxide. The material of the nanowire or at least the surface of the nanowire (for example, coating material) may be, for example but not limited to, ZnO, SiO₂, Li₂O, MgO, Al₂O₃, CaO, TiO₂, Mn₂O₃, Fe₂O₃, CoO, NiO, CuO, Ga₂O₃, SrO, In₂O₃, SnO₂, Sm₂O₃, and EuO and the like.

The nanowires may be grown by a physical vapor deposition method such as pulsed laser deposition, VLS (Vapor-Liquid-Solid) method, CVD (Chemical-Vapor-Deposition) method, arc-discharge method, laser evaporation method, organometallic vapor phase selective growth method, hydrothermal synthetic method, reactive ion etching method, baking method, or melt method.

The nanowires may be charged. The nanowires may have a charge opposite to that of the material to be collected or extracted. Thereby, as a non-limiting example, a charged biomolecule such as an extracellular vesicle, a nucleic acid, or the like can be efficiently attracted or adsorbed.

The nanowires may be fixed to the material forming the flow channel or fluid chamber via another material or member. The material between the nanowires and the wall surface material may have a catalyst for nanowire growth or may be a non-catalytic material.

The nanowires may be grown via a catalyst layer, an adhesion layer, or a growth nucleus. The "layer" may be a thin film. The "layer" may be a continuous film. The "layer" may be discontinuous. The "layer" is a continuous film, and the film may have a hole. The "layer" may be a plurality of separate thin films. The "layer" may be or include islands. The "layer" may be particles and may include particles.

The catalyst layer, adhesion layer, and growth nucleus may be a metal, an alloy, a non-metal, or a semiconductor, or an oxide, a nitride, or the like thereof, or a mixture thereof. The metal includes, but are not limited to, typical elements (alkali metal: Li, Na, K, Rb, Cs, alkaline earth metal: Ca, Sr, Ba, Ra), magnesium group elements: Be, Mg, Zn, Cd, Hg, aluminum group elements: Al, Ga, In, rare earth elements: Y, La, Ce, Pr, Nd, Sm, Eu, tin group elements: Ti, Zr, Sn, Hf, Pb, Th, iron group elements: Fe, Co, Ni, earth acid elements: V, Nb, Ta, chromium group elements: Cr, Mo, W, U, manganese group elements: Mn, Re, noble metals (copper group, coin metal): Cu, Ag, Au, platinum group elements: Ru, Rh, Pd, Os, Ir, Pt, natural radioactive elements: U and Th as a mother radioactive disintegration products: U, Th, Ra, Rn, actinoid, transuranic elements: Np, Pu, Am, Cm, Bk, Cf, Es, Fm, Md, No, and the like, uranium or later elements. The oxide may be any one of them or an oxide of an alloy.

The growth nuclei of the nanowires may be formed of a material different from the wall surface material. The growth nuclei of the nanowires may be formed of a different material than the nanowires. The growth nuclei of the nanowires may be formed of substantially the same material as the wall surface material. The growth nuclei of the nanowires may be, for example, a surface having structural irregularities. The growth nuclei of the nanowires may be, for example, a surface having chemically different properties from part to part. Mechanically, structurally or chemically different (mottled) surfaces may be more susceptible to nanowire growth nuclei in some areas than in others. For example, the irregularities may be formed by lithography and dry wet etching, and the like. For example, ions, neutral atoms, plasma, or the like may be irradiated to form a mechanically, structurally or chemically different (mottled) surface.

The length of the nanowires may be, for example but not limited to, greater than or equal to a value of 500 nm, 1 µm, 1.5 µm, 2 µm, 3 µm, 4 µm, 5 µm, 6 µm, 7 µm, 8 µm, 9 µm, 10 µm, 11 µm, 12 µm, 13 µm, 14 µm, 15 µm, 17 µm, 20 µm, and the like. The length of the nanowires may be, for example, but not limited to, less than or equal to a value of 1 µm, 1.5 µm, 2 µm, 3 µm, 4 µm, 5 µm, 6 µm, 7 µm, 8 µm, 9 µm, 10 µm, 11 µm, 12 µm, 13 µm, 14 µm, 15 µm, 17 µm, 20 µm, 50 µm, 100 µm, 200 µm, and the like.

The diameter of the nanowires (or size in the thickness direction) may be, for example but not limited to, greater than or equal to a value of 5 nm, 10 nm, 15 nm, 20 nm, 25 nm, 30 nm, 40 nm, 50 nm, 60 nm, 70 nm, 80 nm, 90 nm, 100 nm, 150 nm, 200 nm, 250 nm, 300 nm, 400 nm, 500 nm, and the like. The diameter of the nanowires (or size in the thickness direction) may be, for example but not limited to, smaller than or equal to a value of 10 nm, 15 nm, 20 nm, 25 nm, 30 nm, 40 nm, 50 nm, 60 nm, 70 nm, 80 nm, 90 nm, 100 nm, 150 nm, 200 nm, 250 nm, 300 nm, 400 nm, 500 nm, 1 µm, and the like.

The polymer used for the material of the nanowires may be, for example but not limited to, polymethyl methacrylate (PMMA), polystyrene (PS), polydimethylsiloxane (PDMS), conductive polymer poly(3,4-ethylenedioxythiophene)/poly(4-styrenesulfonic acid) (PEDOT/PSS), polyethylene naphthalate (PEN), polyethylene terephthalate (PET), polyimide (PI), or the like.

The nanowires may be a fibrous material and may include a fibrous material. The fibrous material may be a synthetic fiber, may be a natural fiber, and may be a mixture or mixed fibers thereof. The fibrous material may be, for example but not limited to, polyester, polypropylene, polyacrylic, polyamide, a copolymerized polyester-based fiber, polyolefin-based fibers, polyvinyl alcohol-based fiber, and the like. The fibrous material may be, for example but not limited to, a vegetable fiber such as cotton, hemp, hatch, or the like. The fibrous material used for the nanowire may be a woven fabric or a nonwoven fabric. In some embodiments, the nanowires may be a laminate of fibrous materials. In some embodiments, the nanowires may be a structure of short fibers. The length of the short fibers may be random and may have a regularity. The short fiber axes may be randomly arranged or regularly arranged. In some embodiments, the synthetic fibers may be a low melting point material. The low melting point material may be, for example but not limited to, a copolymerized polyester-based fiber, a polyolefin-based fiber, a polyvinyl alcohol-based fiber, or the like. In some embodiments, the synthetic fibers may have a core structure comprising a low melting point polymer.

The spacing between a pair of opposing wall surfaces having nanowires (or the size of the perpendicular direction of the plane on which the nanowires are disposed, hereinafter the same) may be twice the length of the nanowires, may be less than twice, may be 1.5 times, may be more than twice, may be 3 times, 4 times, 5 times, 6 times, 7 times, 8 times, 9 times, 10 times, and may be larger than those.

The spacing between a pair of opposing wall surfaces with nanowires may be less than or equal to 10, 9, 8, 7, 6, 5, 4, 3 times the length of the nanowires.

Hereinafter, some embodiments will be described with reference to the drawings.

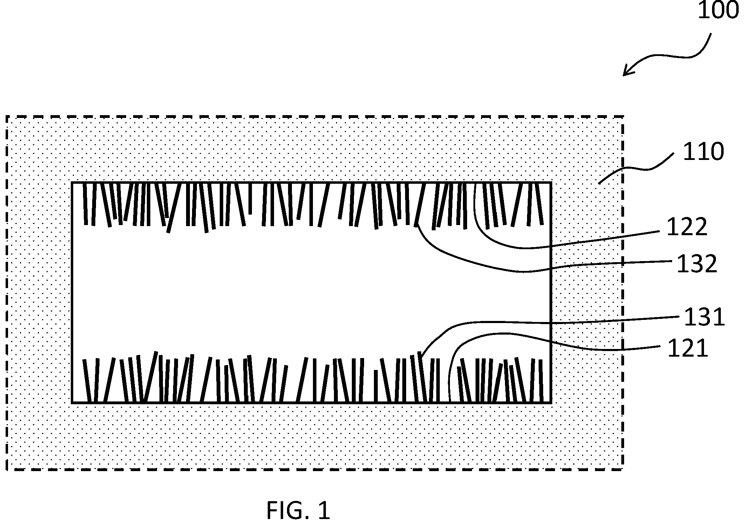

FIG. 1 illustrates a cross-sectional view of a flow channel device (fluid chamber) 100 according to an example not part of the invention. An internal space having a square cross section is formed in the substrate 110 of the flow channel chamber 100 of FIG. 1. Nanowires 131,132 are formed on opposing inner walls 121,122.

In some embodiments, a space may be formed within one member, for example as in FIG. 1. In some embodiments, the internal space may be formed or defined by combining a plurality of members.

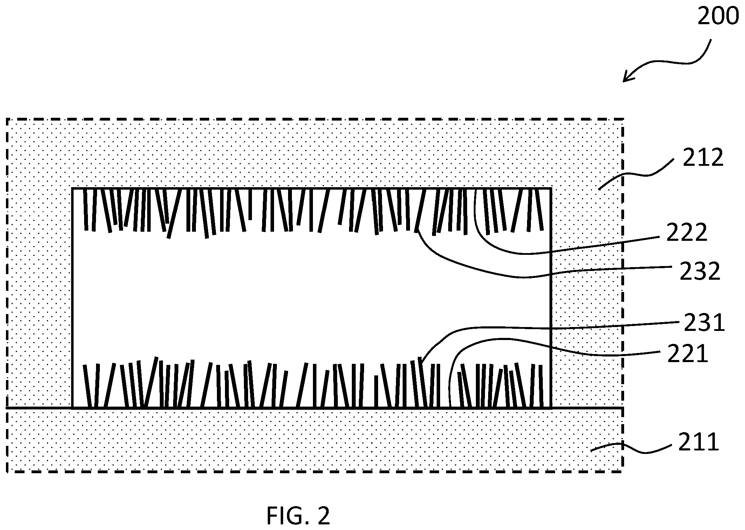

FIG. 2 illustrates a cross-sectional view of a fluid chamber 200 according to an example not part of the invention. The flow channel chamber 200 of FIG. 2 is configured by combining a flat first substrate 211 and a second substrate 212 having a concave portion. The internal space is defined by the combination of these substrates. Nanowires 231 are formed on the inner wall surface 221 of the first substrate 211. Nanowires 232 are formed on the inner wall surface 222 which is located at a position opposite to the inner wall surface 221 of the first substrate and is the bottom surface of the concave portion of the second substrate 212.

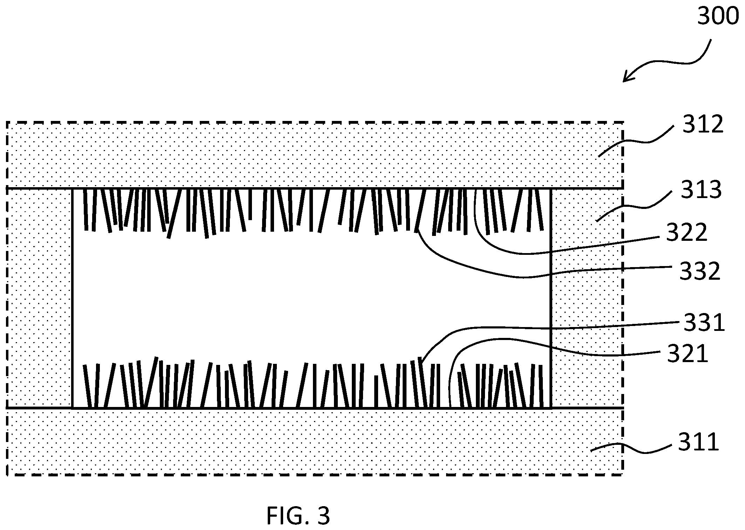

FIG. 3 illustrates a cross-sectional view of a fluid chamber 300 according to one embodiment. The fluid chamber 300 of FIG. 3 is configured by combining a flat first substrate 311 and a flat second substrate 312 with a spacer 313 therebetween. That is, the internal space is defined by the first substrate 311, the second substrate 312 and the spacer 313. Nanowires 331 are formed on the inner wall surface 321 of the first substrate 311. Nanowires 332 are formed on the inner wall surface 322 of the second substrate 312 opposing the inner wall surface 321 of the first substrate 311.

Nanowires may be formed on three or more inner wall surfaces. Nanowires may be formed on all inner wall surfaces defining the fluid chamber.

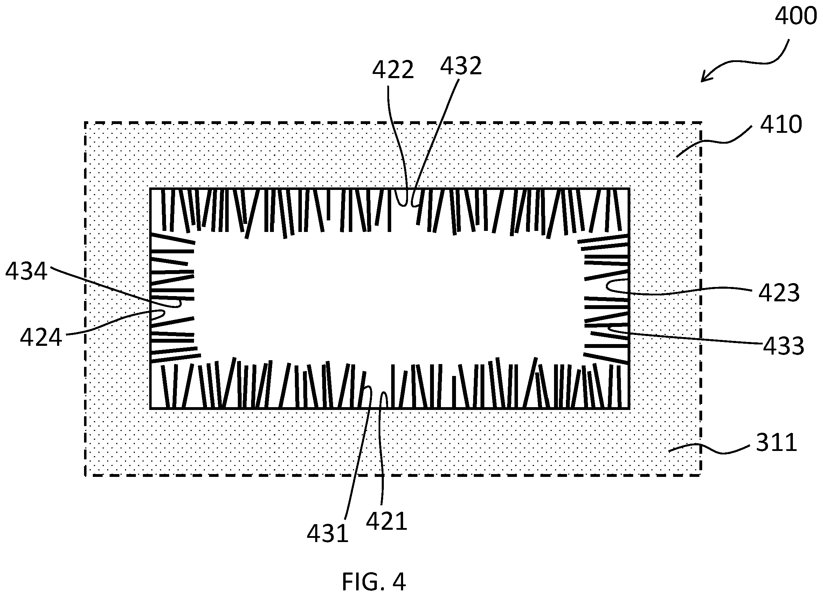

FIG. 4 illustrates a cross-sectional view of a fluid chamber 400 according to an example not part of the invention. The fluid chamber 400 has an internal space formed in the substrate 410, whose cross section has a square shape. The substrate 410 has inner walls 421,422,423,424, and an internal space is defined by these inner walls. In FIG. 4, nanowires 431,432,433,434 are formed on each of these inner walls 421,422,423,424.

In some embodiments, it may not be possible to define the number of inner walls that constitute the internal space. In some embodiments, the internal space may be formed with a curved surface.

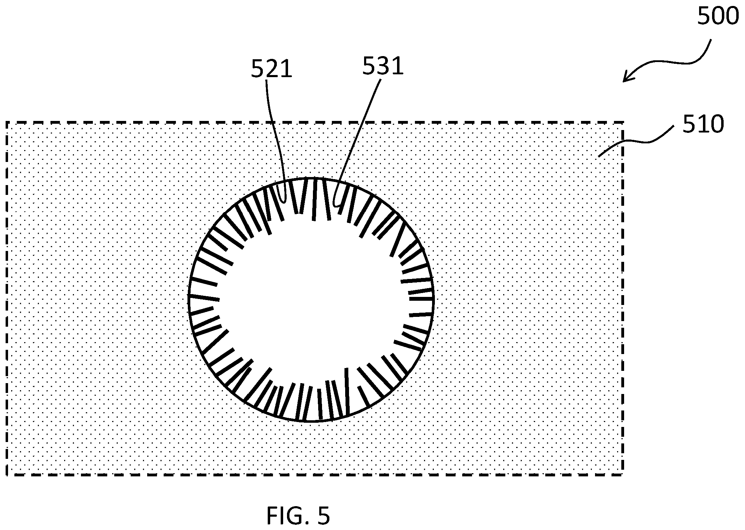

FIG. 5 illustrates a cross-sectional view of a fluid chamber 500 according to an example not part of the invention. The fluid chamber 500 has an internal space that is circular in cross-section in the substrate 510. Nanowires 531 are formed on the inner wall 521 of the curved surface (spherical or cylindrical inner surface).

In some embodiments, one or more inner walls may have unevenness.

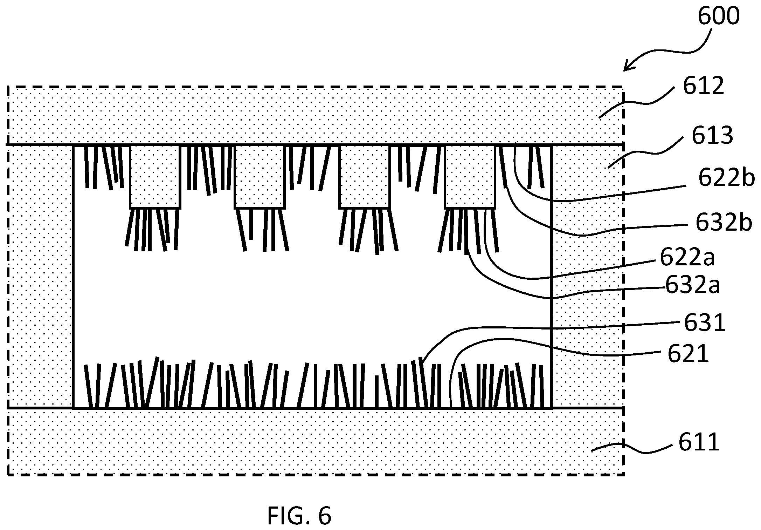

FIG. 6 illustrates a cross-sectional view of a fluid chamber 600 according to one embodiment. The fluid chamber 600 is configured by combining a flat first substrate 611 and a macroscopically flat second substrate 612 having unevenness on the inner wall, and a spacer 613 therebetween. Nanowires 631 are formed on the inner wall 621 of the first substrate 611. The inner wall 622 of the second substrate 612 has a convex portion 622a and a concave portion or bottom surface 622b. Nanowires 632a are formed on the convex portion 622a, and nanowires 632b are also formed on the bottom surface 622b.

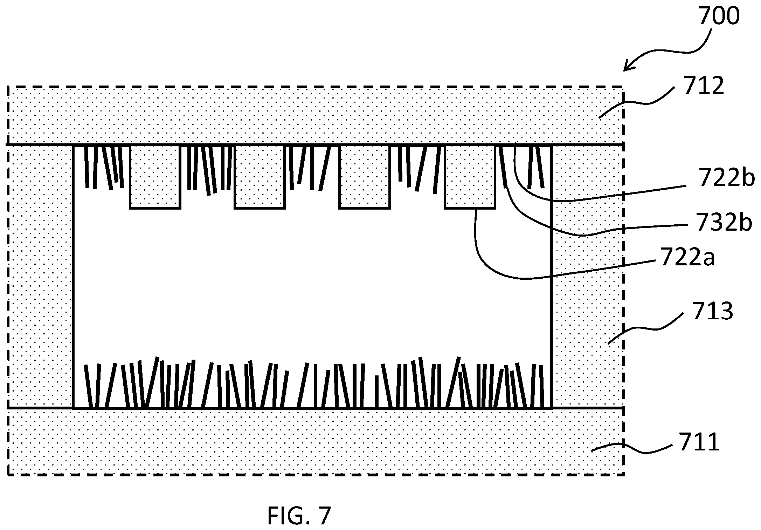

FIG. 7 illustrates a cross-sectional view of a fluid chamber 700 according to one embodiment. The fluid chamber 700 is configured by combining a flat first substrate 711 and a macroscopically flat second substrate 712 having unevenness on the inner wall, and a spacer 713 therebetween. No nanowires are formed on the convex portion 722a, and nanowires 732 are formed on the bottom surface 722b.

In some embodiments, the nanowires may be formed on all of the inner wall surfaces having unevenness, may not be formed on all of them, the nanowires may be formed on a part of the irregularity surface. In the fluid chamber 600 shown in FIG. 6, nanowires 632a,632b are formed on the surface 622a facing the internal space of the convex portion and on a concave portion or bottom surface 622b. In the fluid chamber 700 shown in FIG. 7, nanowires 732 are formed in the concave portion 722b. In some embodiments, nanowires may be formed on the lateral surfaces of the unevenness (not shown).

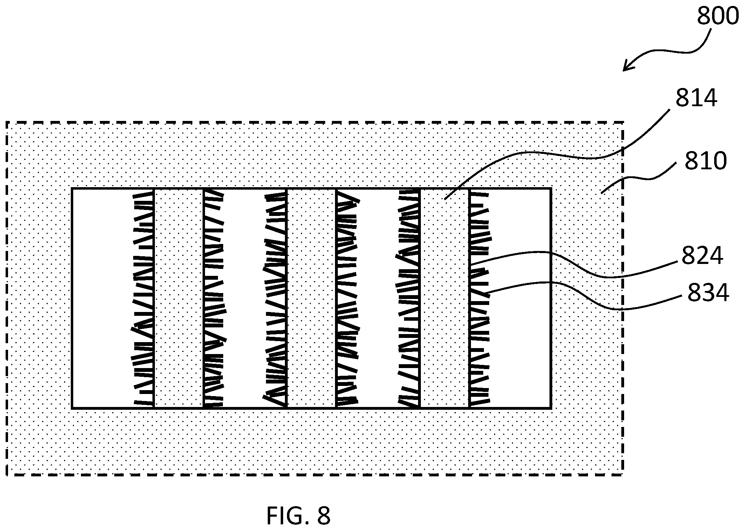

FIG. 8 illustrates a cross-sectional view of a fluid chamber 800 according to an example not part of the invention. The fluid chamber 800 has a structure 814 in the internal space formed in the substrate 810, which is separate from the inner wall defining the internal space. The structure 814 is configured continuously from one inner wall to another or to an opposing inner wall. Nanowires 834 are formed on the surface 824 of the structure 814 (which may be referred to as an inner wall).

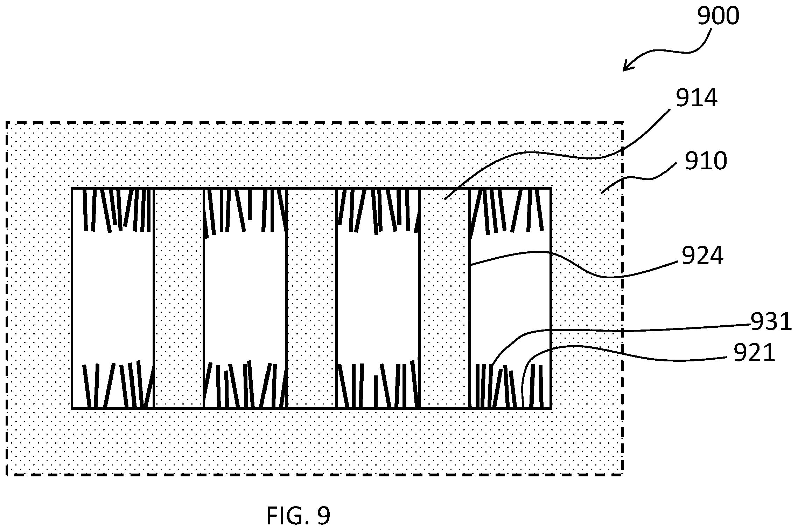

FIG. 9 illustrates a cross-sectional view of a fluid chamber 900 according to an example not part of the invention. The fluid chamber 900 has a structure 914 in the internal space formed in the substrate 910, which is separate from the inner wall defining the internal space. In the fluid chamber 900 shown in FIG. 9, no nanowires are formed on the surface 924 of the structure 914. Nanowires 931 are formed on the inner wall 921.

In addition to the inner walls defining the outer frame of the internal space, an uneven portion or a structure may be disposed. All of the surfaces of these uneven portions and structures and the inner walls defining the outer frame of the internal space may be referred to as inner walls. The surfaces of the inner walls defining the outer frame of the internal space, the surfaces of the uneven portion and the structure may be defined as other inner walls.

Nanowires may be formed on all of the surfaces of these uneven portions and structures and the inner walls defining the outer frame of the internal space or nanowires may be formed on a part thereof. Nanowires may be formed on all the surfaces of the inner walls, or nanowires may be formed on a part or all of the surface.

In some embodiments, the unevenness or structures disposed in the internal space may be so-called chaotic mixers and may have a structure that causes non-linear and/or three-dimensional flow of the fluid flowing through the internal space. Such a structure may have, for example, a change in step or cross-sectional area in the flow channel, such as a change in the direction of the flow channel.

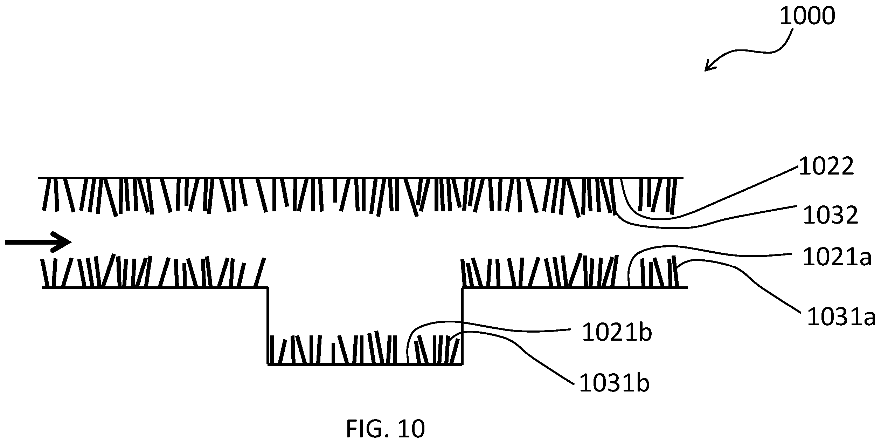

FIG. 10 illustrates a cross-sectional view of a fluid chamber 1000 according to an example not part of the invention. The fluid chamber 1000 may be a flow channel. The solution flows in the direction of the arrow in FIG. 10. The flow channel 1000 has opposing inner walls 1021,1022. The inner wall 1021 has a concave portion 1021. Due to the step between the inner wall surface 1021a and the concave portion 1021b which have flowed in the direction of the arrow, the direction of flow is changed and the flow becomes nonlinear. This is believed to increase the probability that the material in the solution will contact with or reach near the nanowires 1031,1032, for example.

The structure for providing a step, or a structure that gives variation in the cross-sectional area, and the change in the direction of the flow channel may be provided on one inner wall, or may be provided on at least one inner wall or a plurality of the inner walls.

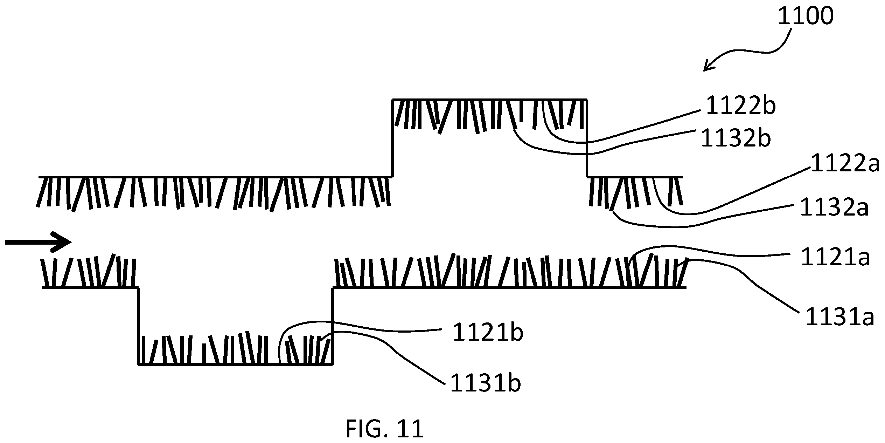

FIG. 11 illustrates a cross-sectional view of a fluid chamber 1100 according to an example not part of the invention. The fluid chamber 11 has steps provided on the opposing inner walls. Concave portions 1121b,1122b are disposed on the opposing inner walls 1121a,1122a respectively, so as to be shifted in the flow path direction. Nanowires 1131a,1131b,1132a 1132b are also disposed on the normal inner wall surfaces 1121a,1122a and the concave portions 1121b,1122b. This is believed to increase the probability that materials in the solution flowing in the direction of the arrows will contact with or reach near the nanowires, for example.

The uneven structure or a structure body such as chaotic mixer may take on a variety of configurations. For example, a concave portion may be formed in the inner wall. The concave portion may be formed in a stripe shape (groove). The concave portion may be formed as a plurality of parallel stripes. The stripe-shaped concave portions may be parallel to or angled with respect to the direction in which the solution flows. The angle may be substantially vertical or may be between 0 and 90 degrees.

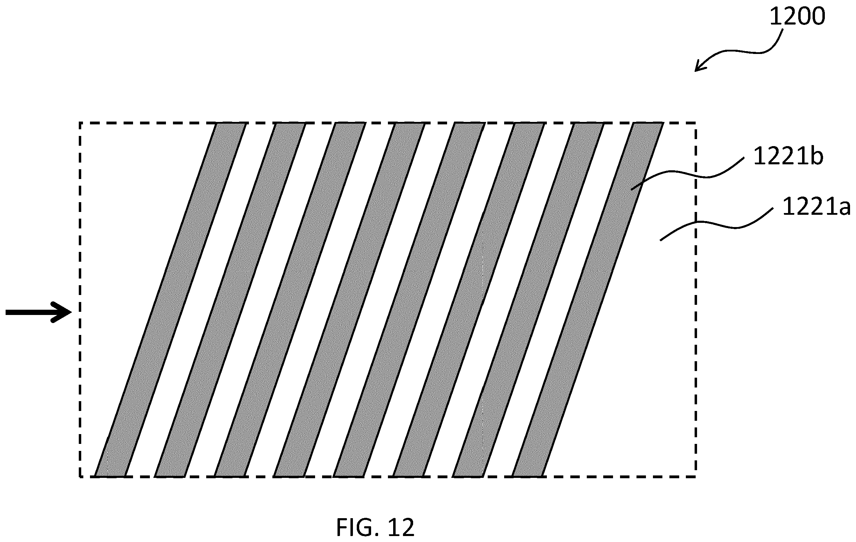

FIG. 12 illustrates a top view of one inner wall of the flow channel 1200 according to an example not part of the invention. On the inner wall 1221a, concave portions or grooves 1221b are repeatedly formed in parallel in a stripe shape. The concave portion 1221b is disposed such that the longitudinal direction thereof has an angle with respect to the flow direction of the solution indicated by an arrow.

The uneven structure or a structure body such as chaotic mixer may be linear or bent.

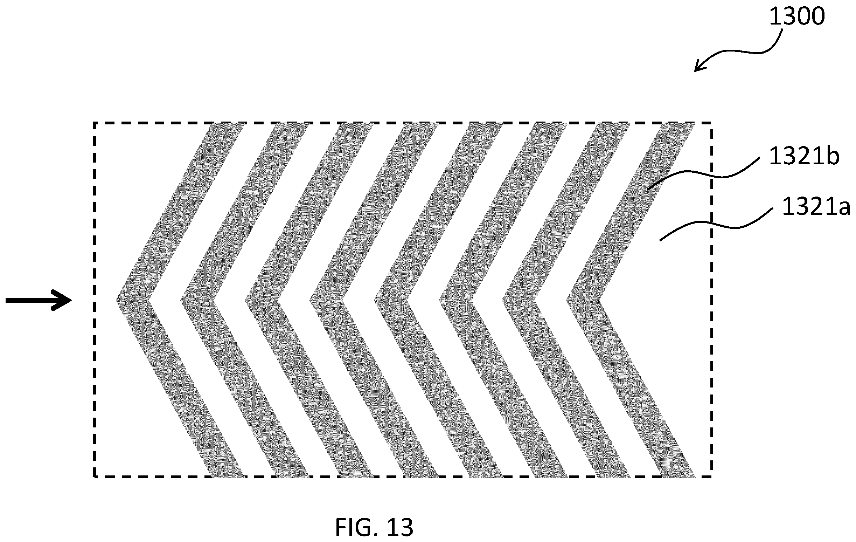

FIG. 13 illustrates a top view of one inner wall of the flow channel 1300 according to an example not part of the invention. On the inner wall 1321a, concave portions or grooves 1321b are formed in a stripe shape, partially bent downward, and repeating in parallel with each other. The concave portion 1321b is disposed such that the longitudinal direction thereof has an angle with respect to the flow direction of the solution indicated by an arrow. Such an arrangement may be referred to as a herringbone shape.

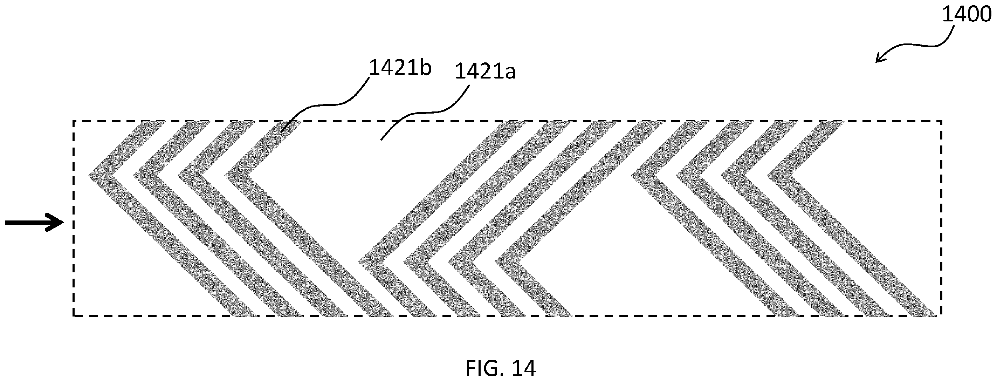

FIG. 14 illustrates a top view of one inner wall of the flow channel 1400 according to an example not part of the invention. On the inner wall 1421a, structures in which concave portions or grooves 1421b are partially bent in a stripe shape are continuously formed while the bent portions are alternately shifted.

A chaotic mixer having unevenness in a herringbone shape on an inner wall surface of a flow channel can promote nonlinear flow of a fluid. Thus, by way of example, nanowires disposed on a plurality of inner wall surfaces or curved inner wall surfaces can trap more biomolecules in the solution.

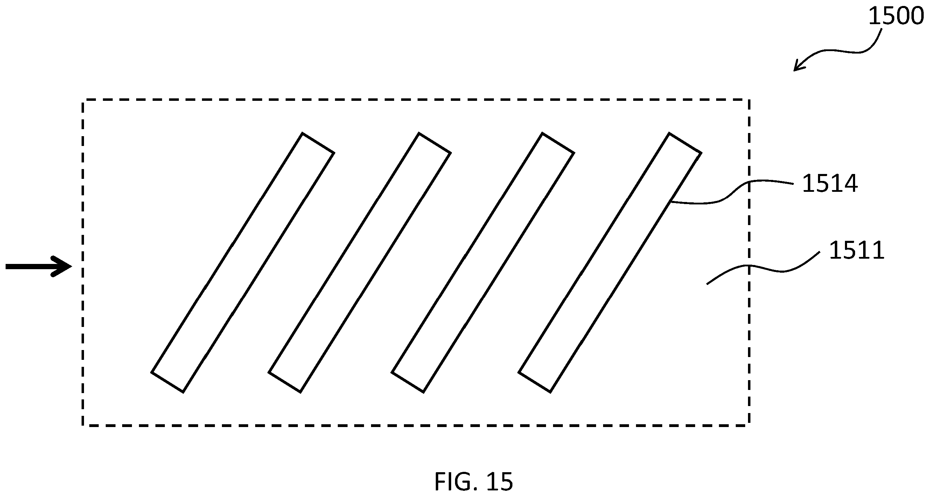

FIG. 15 illustrates a top view of one inner wall of the flow channel 1500 according to an example not part of the invention. On the inner wall 1511, the structures (walls) 1514 are formed repeatedly in parallel. The walls 1514 are disposed such that the longitudinal direction is angled relative to the direction in which the solution flows, as indicated by an arrow.

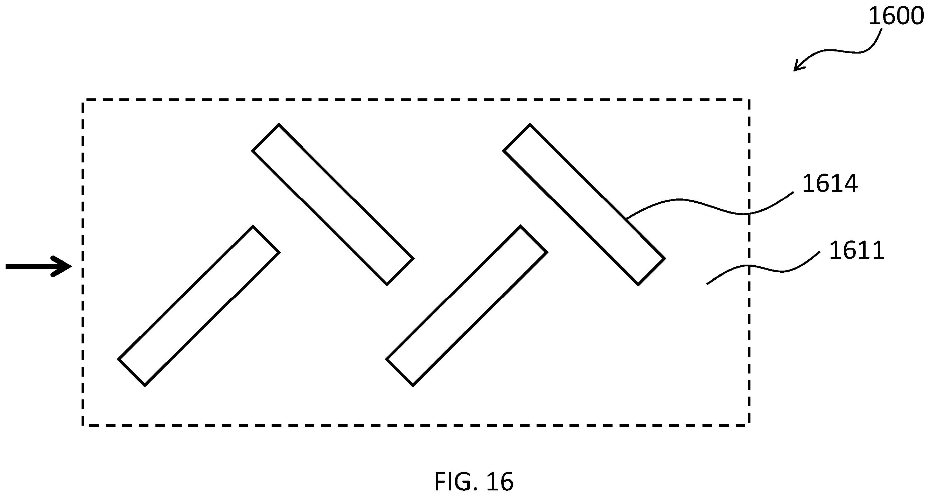

FIG. 16 illustrates a top view of one inner wall of the flow channel 1600 according to an example not part of the invention. On the inner wall 1611, the structures (walls) 1614 are formed alternately and repeatedly. The walls 1614 are disposed such that the longitudinal direction is angled relative to the direction in which the solution flows, as indicated by an arrow.

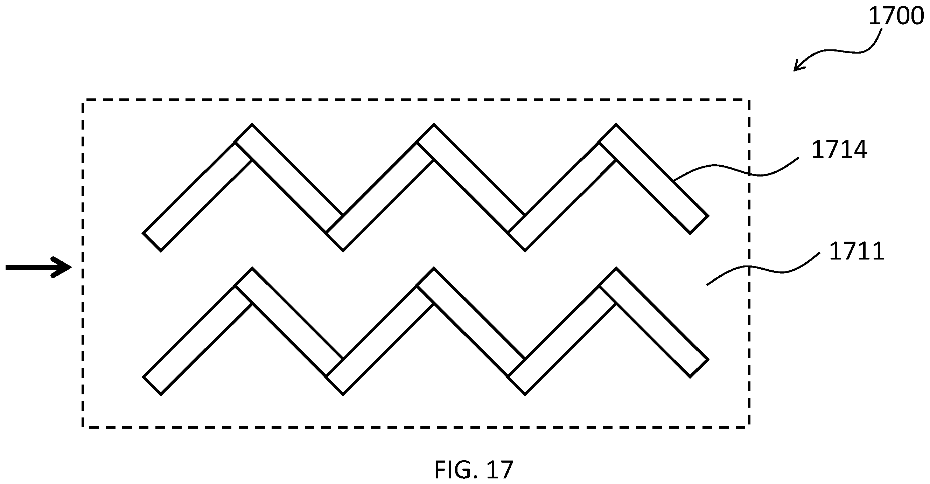

FIG. 17 illustrates a top view of one inner wall of the flow channel 1700 according to an example not part of the invention. On the inner wall 1711, zigzag-shaped structures (walls) 1714 are formed.

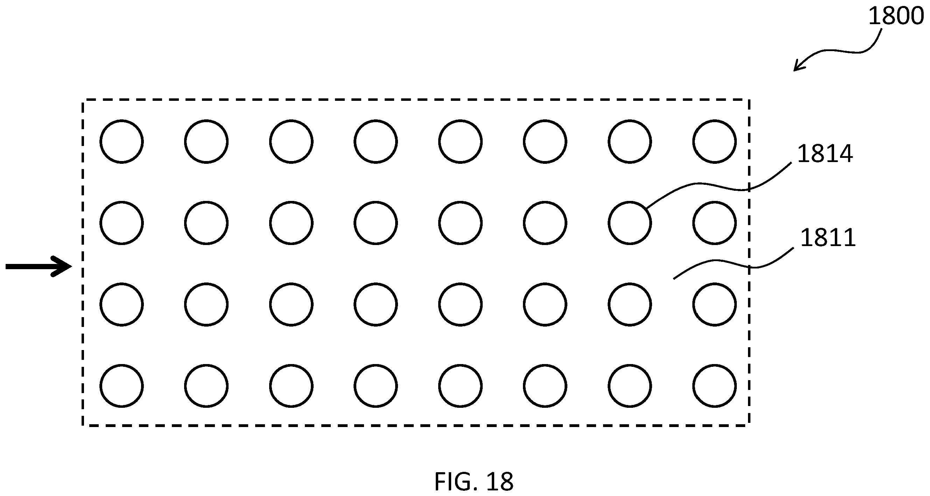

FIG. 18 illustrates a top view of one inner wall of the flow channel 1800 according to an example not part of the invention. On the inner wall 1811, the structures (pillars) 1814 are disposed in a grid shape along the flow channel direction of an arrow.

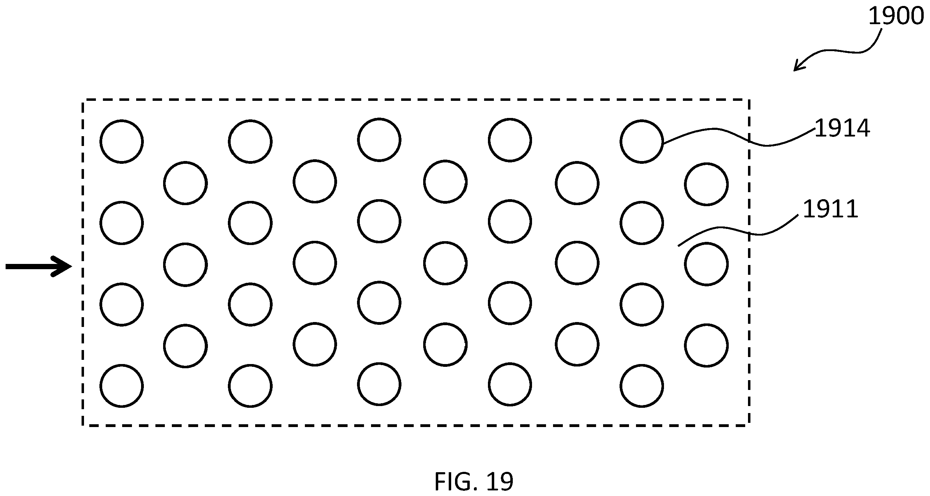

FIG. 19 illustrates a top view of one inner wall of the flow channel 1900 according to an example not part of the invention. On the inner wall 1911, pillars 1914 are disposed staggered from each other in the flow channel direction of the arrow.

Structures such as walls and pillars disposed on the inner wall surface of the flow channel may be formed continuously to the opposing inner walls, or may not be continuous to the opposing walls and may have an end in the internal space. These structures are capable of agitating the flowing solution. Thus, by way of example, nanowires disposed on a plurality of inner wall surfaces or curved inner wall surfaces can trap more biomolecules in the solution.

The flow channel may be straight, bent, or curved.

In some embodiments, the fluid chamber or flow channel device may be connected to or configured to be connected to an analysis device. In some embodiments, the fluid chamber or flow channel device may be incorporated in an analysis device. The analysis device may be, for example but not limited to, an analytical or measuring device, such as optical, magnetic, electrical, chemical, electrochemical, or the like. In some embodiments, the analysis device may be a nucleic acid (RNA, DNA) sequencer. In some embodiments, it may be a microarray.

The present disclosure includes a method of collecting, extracting or accumulating biomolecules (also simply referred to as a collection method). In some embodiments, the collection method may include introducing a solution into a fluid chamber or flow channel (hereinafter also referred to as a fluid chamber) or bringing the solution into contact with the nanowires.

In some embodiments, the introduction of a solution into a fluid device may cause the solution to rest substantially in the fluid device after introducing the solution. In some embodiments, the introduction of a solution into the fluid device may continue to flow the solution continuously into the fluid device. For example, it may be continued to introduce a solution from an inlet of a flow channel device and discharge the solution that has passed through the fluid channel device from the outlet. For example, the solution may be in contact with the nanowires while constantly flowing in the fluid device.

In collecting charged molecules such as microRNAs, the nanowires may have a positive surface charge. For example, the nanowires may be contacted with body fluids under pH conditions where the nanowires have a positive surface charge. This allows, for example, free and EV inclusive forms of microRNA to be captured on the nanowires. In some embodiments, the pH of the body fluid may be adjusted such that the nanowires have a positive surface charge. In some embodiments, the nanowires may be made of a material or method having a positive surface charge to match the pH of the solution.

In some embodiments, the collection method may include adjusting the pH of the solution. The pH of the solution may be adjusted before, after, or during contact with the nanowires. In some embodiments, the pH of the body fluid may be adjusted to be greater than or equal to a value such as 2, 3, 4, or 5. In some embodiments, the pH of the body fluid may be adjusted to be less than or equal to a value such as 10, 9, 8, 7,6, or 5. In some embodiments, the pH of urine may be adjusted to 6 to 8.

In some embodiments, in the collection, a dissociating agent (or a releaser, a dissociating solution, a solution for dissociating, or the like) may be introduced after introduction of the solution into the fluid device. This allows, for example, molecules captured by the nanowire or in the fluid device to dissociate from the nanowires. In some embodiments, the collection method may include collecting captured material along with the dissociating agent. The dissociating agent may include a buffering agent. In some embodiments, the dissociating agent may comprise a surfactant. The surfactant may be, for example, a nonionic surfactant and may be an ionic surfactant. This allows, for example, the RNA contained in the EV captured by the nanowires or the RNA in free form in solution and captured by the nanowires to dissociate from the nanowire. In some embodiments, the dissociating agent may include a RNase inhibitor.

In some embodiments, the collection method may include washing after introduction of the solution into the fluid device. In some embodiments, the collection method may perform washing prior to introduction of the releaser. Washing may include introducing water, a buffer, a washing liquid, or the like (simply referred to as a washing liquid) into a fluid device. The washing may cause the washing liquid after the washing to be discharged. Thus, substances other than the substance captured by the nanowires (solution or molecules) can be discharged out of the fluid device, for example but in a non-limiting manner. In some embodiments, washing may not be performed. For example, it may be non-washed.

The present disclosure also includes methods of measuring and analyzing collected molecules. In some embodiments, biomolecules collected in a fluid device may be analyzed. In some embodiments, the amount of expression of RNA in a body fluid may be analyzed. The RNA may be microRNA. In some embodiments, the expression profile of RNA collected in a fluidic device may be measured using a microarray or sequencer. The measurement may include introducing a solution containing the collected RNA into a microarray or sequencer.

The present disclosure also includes diagnostic methods. In some embodiments, the diagnosis of a disease, the risk of a disease, or the like may be performed on the basis of the expression profile of the RNA collected in a fluid device, the amount of expression of one or more specific RNAs, or temporal changes thereof.

The present disclosure includes a program or software for performing a measurement method, an analysis method, and a diagnostic method. The program or the software may be recorded in a storage medium. The method may include transmitting the expression profile of RNA collected in the fluid device, or the amount of expression of one or more specific RNAs, to a computing device, such as a PC, server, CPU, and the like. It may include receiving the expression profile of the RNA collected in a fluidic device, or the amount of expression of one or more specific RNAs. Receiving and transmitting may be performed by wire, wirelessly, or transmitted via the Internet. Saving, storage, and transmission and reception of data may be performed via a cloud. Analysis and diagnosis may be performed using artificial intelligence, machine learning, depth learning, or the like.

While several embodiments and examples of the present disclosure have been described above, these embodiments and examples illustratively explain the present disclosure. For example, each of the embodiments described above has been described in detail in order to explain the present disclosure in an easy-to-understand manner, and dimensions, configurations, materials, and circuits may be additionally changed as necessary. Embodiments in which one or more features of the present disclosure described above are arbitrarily combined are also included in the scope of the present disclosure. Accordingly, the embodiments and examples disclosed herein have been shown by way of illustration and should not be considered as limiting the scope of the present disclosure. The scope of the present invention is limited only by the scope of the appended claims.

### DESCRIPTION OF REFERENCES

100 Flow channel device (fluid chamber)
110 Substrate
121,122 Inner wall
131,132 Nanowires
200 Fluid chamber
211 First substrate
212 Second substrate
221 Inner wall surface
231 Nanowires
222 Inner wall surface
232 Nanowires
300 Fluid chamber
311 First substrate
312 Second substrate
321 Inner wall surface
331 Nanowires
322 Inner wall surface
332 Nanowires
400 Fluid chamber
410 Substrate
421,422,423,424 Inner wall
431,432,433,434 Nanowires
500 Fluid chamber
510 Substrate
521 Inner wall
531 Nanowires
600 Fluid chamber
611 First substrate
612 Second substrate
613 Spacer
621 Inner wall
631 Nanowires
612 Second substrate
622 Inner wall
622a Convex portion
622b Concave portion or bottom surface
632a Nanowires
632b Nanowires
700 Fluid chamber
711 First substrate
712 Second substrate
713 Spacer
722a Convex portion
722b Bottom surface
732 Nanowires
800 Fluid chamber
810 Substrate
814 Structure
824 Surface (inner wall)
834 Nanowires
900 Fluid chamber
910 Substrate
914 Structure
924 Structure
921 Inner wall
931 Nanowires
1000 Fluid chamber
10,211,022 Inner wall
1021 Concave portion
1021a inner wall surface
1021b Concave portion
1031,1032 Nanowires
1100 Fluid chamber
1121a, 1122a Inner walls
1121b, 1122b Concave portion
1131a,1131b,1132a,1132b Nanowires
1200 Flow channel
1221a Inner wall
1221b Concave portion or groove
1300 Flow channel
1321a Inner wall
1321b Concave portion or groove
1400 Flow channel
1421a Inner wall
1421b Concave portion or groove
1500 Flow channel
1511 Inner wall
1514 Structure (wall)
1600 Flow channel
1611 Inner wall
1614 Structure (wall)
1700 Flow channel
1711 Inner wall
1714 Structure (wall)
1800 Flow channel
1811 Inner wall
1814 Structure (pillar)
1900 Flow channel
1911 Inner wall
1914 Structure (pillar)

## Claims

1. A biomolecule collection device, comprising:
a fluid chamber (300, 600, 700) that is at least partially rectangular parallelepiped;
nanowires (331, 332, 631, 632a, 632b, 732) disposed on both of opposing inner walls (321, 322, 621, 622) of at least one pair of the rectangular parallelepiped walls of the fluid chamber (300, 600, 700), wherein the fluid chamber (300, 600, 700) is **characterized in that** it comprises:
a pair of substrates (311, 312, 611, 612, 711, 712) having a substantially flat surface and having nanowires (331, 332, 631, 632a, 632b, 732) disposed on the substantially flat surface, the pair of substrates (110) being bonded so that the surfaces on which the nanowires (331, 332, 631, 632a, 632b, 732) are disposed oppose each other; and
a spacer (313, 613, 713) sandwiched between the pair of substrates (311, 312, 611, 612, 711, 712); the spacer (313, 613, 713) configured to define a space having the nanowires (331, 332, 631, 632a, 632b, 732) between the opposing surfaces of the pair of substrates (311, 312, 611, 612, 711, 712).

2. The biomolecule collection device according to claim 1,
wherein at least one of the plurality of inner walls (321, 322, 621, 622) has a uneven structure.

3. The biomolecule collection device according to any one of claim 1 or 2,
wherein the fluid chamber (300, 600, 700) has an inlet for introducing a solution containing the biomolecules, and an outlet for discharging it, the fluid chamber (300, 600, 700) being configured as a flow channel in which the solution flows.

4. The biomolecule collection device according to any one of claims 1 to 3,
wherein the fluid chamber (300, 600, 700) includes a chaotic mixer.

5. The biomolecule collection device according to claim 4,
wherein the nanowires (331, 332, 631, 632a, 632b, 732) are disposed on at least a portion of a surface of the chaotic mixer; or wherein the nanowires (331, 332, 631, 632a, 632b, 732) are disposed directly on a surface on which the nanowires (331, 332, 631, 632a, 632b, 732) are disposed; or wherein the nanowires (331, 332, 631, 632a, 632b, 732) are embedded at one end thereof in an inner wall (321, 322, 621, 622) on which the nanowires (331, 332, 631, 632a, 632b, 732) are disposed.

6. A biomolecule collection device according to any one of claims 1 to 4,
wherein the inner wall (321, 322, 621, 622) on which the nanowires (331, 332, 631, 632a, 632b, 732) are disposed has a growth layer, and
wherein the nanowires (331, 332, 631, 632a, 632b, 732) are formed by growing on the growth layer.

7. The biomolecule collection device according to claim 6,
wherein the growth layer includes a catalyst for growing the nanowires (331, 332, 631, 632a, 632b, 732).

8. A biomolecule analysis device comprising said biomolecule collection device according to any one of claims 1 to 7.

9. A method for collecting biomolecules, comprising:
providing the biomolecule collection device according to claims 1 to 7; and
introducing a solution containing a biomolecule into the biomolecule collection device.

10. A method for collecting biomolecules according to claim 9,
wherein said introducing the solution including the biomolecule into the biomolecule collection device is continuously introducing the solution including the biomolecule.

11. The method according to claim 9 or 10, further comprising introducing a dissociating agent into the biomolecule collection device to dissociate the captured biomolecule from the nanowire.

12. The method according to any one of claims 9 to 11,
wherein the biomolecule includes a microRNA.

13. The method according to claim 12,
wherein the solution is urine or saliva.

14. A method of analyzing RNA expression, comprising:
providing the biomolecule analysis device according to claim 8 and using the biomolecule collection device **to** collect RNA; measuring the collected RNA using the biomolecule analysis device; and
estimating the amount of expression of the RNA in the body fluid based on the measured RNA data.

15. The method according to claim 14,
wherein said estimating the amount of expression of the RNA in the body fluid includes determining an expression profile of the RNA in the body fluid.

16. The method according to claim 14 or 15, wherein the body fluid is urine or saliva.

## Patentansprüche

1. Biomolekül-Sammelvorrichtung, umfassend:
eine Fluidkammer (300, 600, 700), die zumindest teilweise rechtwinklige parallelepipedische Form aufweist;
Nanodrähte (331, 332, 631, 632a, 632b, 732), die auf beiden gegenüberliegenden Innenwänden (321, 322, 621, 622) von mindestens einem Paar der rechtwinkligen parallelepipedischen Wände der Fluidkammer (300, 600, 700) angeordnet sind, wobei die Fluidkammer (300, 600, 700) **dadurch gekennzeichnet ist, dass** sie Folgendes umfasst:
ein Paar von Substraten (311, 312, 611, 612, 711, 712) mit einer im Wesentlichen flachen Oberfläche und mit Nanodrähten (331, 332, 631, 632a, 632b, 732), die auf der im Wesentlichen flachen Oberfläche angeordnet sind, wobei das Paar von Substraten (110) so zusammengefügt ist, dass die Oberflächen, auf denen die Nanodrähte (331, 332, 631, 632a, 632b, 732) angeordnet sind, einander gegenüberliegen; und
einen Abstandshalter (313, 613, 713), der zwischen dem Paar von Substraten (311, 312, 611, 612, 711, 712) sandwichartig eingefasst ist; wobei der Abstandshalter (313, 613, 713) dazu ausgelegt ist, einen Raum mit den Nanodrähten (331, 332, 631, 632a, 632b, 732) zwischen den gegenüberliegenden Oberflächen des Paars von Substraten (311, 312, 611, 612, 711, 712) zu definieren.

2. Biomolekül-Sammelvorrichtung nach Anspruch 1, wobei mindestens eine von der Vielzahl von Innenwänden (321, 322, 621, 622) eine unebene Struktur aufweist.

3. Biomolekül-Sammelvorrichtung nach einem der Ansprüche 1 oder 2,
wobei die Fluidkammer (300, 600, 700) einen Einlass zum Einführen einer Lösung, die die Biomoleküle enthält, und einen Auslass zum Abführen davon aufweist, wobei die Fluidkammer (300, 600, 700) als ein Strömungskanal konfiguriert ist, in dem die Lösung fließt.

4. Biomolekül-Sammelvorrichtung nach einem der Ansprüche 1 bis 3,
wobei die Fluidkammer (300, 600, 700) einen chaotischen Mischer beinhaltet.

5. Biomolekül-Sammelvorrichtung nach Anspruch 4,
wobei die Nanodrähte (331, 332, 631, 632a, 632b, 732) auf mindestens einem Teil einer Oberfläche des chaotischen Mischers angeordnet sind; oder wobei die Nanodrähte (331, 332, 631, 632a, 632b, 732) direkt auf einer Oberfläche angeordnet sind, auf der die Nanodrähte (331, 332, 631, 632a, 632b, 732) angeordnet sind; oder wobei die Nanodrähte (331, 332, 631, 632a, 632b, 732) an einem Ende davon in einer Innenwand (321, 322, 621, 622) eingebettet sind, auf der die Nanodrähte (331, 332, 631, 632a, 632b, 732) angeordnet sind.

6. Biomolekül-Sammelvorrichtung nach einem der Ansprüche 1 bis 4,
wobei die Innenwand (321, 322, 621, 622), auf der die Nanodrähte (331, 332, 631, 632a, 632b, 732) angeordnet sind, eine Wachstumsschicht aufweist, und
wobei die Nanodrähte (331, 332, 631, 632a, 632b, 732) durch Aufwachsen auf der Wachstumsschicht gebildet werden.

7. Biomolekül-Sammelvorrichtung nach Anspruch 6,
wobei die Wachstumsschicht einen Katalysator für das Aufwachsen der Nanodrähte (331, 332, 631, 632a, 632b, 732) beinhaltet.

8. Biomolekül-Analysevorrichtung, umfassend die Biomolekül-Sammelvorrichtung nach einem der Ansprüche 1 bis 7.

9. Verfahren zum Sammeln von Biomolekülen, umfassend:
Bereitstellen der Biomolekül-Sammelvorrichtung nach den Ansprüchen 1 bis 7; und
Einführen einer Lösung, die ein Biomolekül enthält, in die Biomolekül-Sammelvorrichtung.

10. Verfahren zum Sammeln von Biomolekülen nach Anspruch 9,
wobei das Einführen der Lösung, die das Biomolekül beinhaltet, in die Biomolekül-Sammelvorrichtung ein kontinuierliches Einführen der Lösung, die das Biomolekül beinhaltet, ist.

11. Verfahren nach Anspruch 9 oder 10, ferner umfassend Einführen eines Dissoziationsmittels in die Biomolekül-Sammelvorrichtung, um das eingefangene Biomolekül von dem Nanodraht zu dissoziieren.

12. Verfahren nach einem der Ansprüche 9 bis 11,
wobei das Biomolekül eine microRNA beinhaltet.

13. Verfahren nach Anspruch 12,
wobei die Lösung Urin oder Speichel ist.

14. Verfahren zum Analysieren der RNA-Expression, umfassend:
Bereitstellen der Biomolekül-Analysevorrichtung nach Anspruch 8 und Verwenden der Biomolekül-Sammelvorrichtung zum Sammeln von RNA;
Messen der gesammelten RNA unter Verwendung der Biomolekül-Analysevorrichtung; und
Schätzen der Expressionsmenge der RNA in der Körperflüssigkeit basierend auf den gemessenen RNA-Daten.

15. Verfahren nach Anspruch 14,
wobei das Schätzen der Expressionsmenge der RNA in der Körperflüssigkeit das Bestimmen eines Expressionsprofils der RNA in der Körperflüssigkeit beinhaltet.

16. Verfahren nach Anspruch 14 oder 15, wobei die Körperflüssigkeit Urin oder Speichel ist.

## Revendications

1. Dispositif de collecte de biomolécules, comprenant :
une chambre de fluide (300, 600, 700) qui est un parallélépipède au moins partiellement rectangulaire ;
des nanofils (331, 332, 631, 632a, 632b, 732) disposés sur les deux parois internes opposées (321, 322, 621, 622) d'au moins une paire de parois de parallélépipède rectangulaire de la chambre de fluide (300, 600, 700), la chambre de fluide (300, 600, 700) étant **caractérisée en ce qu'**elle comprend :
une paire de substrats (311, 312, 611, 612, 711, 712) ayant une surface sensiblement plate et comportant des nanofils (331, 332, 631, 632a, 632b, 732) disposés sur la surface sensiblement plate, la paire de substrats (110) étant liée de sorte que les surfaces sur lesquelles les nanofils (331, 332, 631, 632a, 632b, 732) sont disposés se fassent face ; et
un élément d'espacement (313, 613, 713) intercalé entre la paire de substrats (311, 312, 611, 612, 711, 712) ;
l'élément d'espacement (313, 613, 713) étant configuré pour définir un espace comportant les nanofils (331, 332, 631, 632a, 632b, 732) entre les surfaces opposées de la paire de substrats (311, 312, 611, 612, 711, 712).

2. Dispositif de collecte de biomolécules selon la revendication 1,
dans lequel au moins l'une de la pluralité de parois internes (321, 322, 621, 622) a une structure irrégulière.

3. Dispositif de collecte de biomolécules selon l'une quelconque des revendications 1 ou 2,
dans lequel la chambre de fluide (300, 600, 700) comporte une entrée pour introduire une solution contenant les biomolécules et une sortie pour évacuer celle-ci, la chambre de fluide (300, 600, 700) étant configurée sous la forme d'un canal d'écoulement dans lequel la solution s'écoule.

4. Dispositif de collecte de biomolécules selon l'une quelconque des revendications 1 à 3,
la chambre de fluide (300, 600, 700) comprend un mélangeur chaotique.

5. Dispositif de collecte de biomolécules selon la revendication 4,
dans lequel les nanofils (331, 332, 631, 632a, 632b, 732) sont disposés sur au moins une partie d'une surface du mélangeur chaotique ; ou dans lequel les nanofils (331, 332, 631, 632a, 632b, 732) sont disposés directement sur une surface sur laquelle les nanofils (331, 332, 631, 632a, 632b, 732) sont disposés ; ou dans lequel les nanofils (331, 332, 631, 632a, 632b, 732) sont incorporés à une extrémité de ceux-ci dans une paroi interne (321, 322, 621, 622) sur laquelle les nanofils (331, 332, 631, 632a, 632b, 732) sont disposés.

6. Dispositif de collecte de biomolécules selon l'une quelconque des revendications 1 à 4,
dans lequel la paroi interne (321, 322, 621, 622) sur laquelle les nanofils (331, 332, 631, 632a, 632b, 732) sont disposés comporte une couche de croissance, et
dans lequel les nanofils (331, 332, 631, 632a, 632b, 732) sont formés par croissance sur la couche de croissance.

7. Dispositif de collecte de biomolécules selon la revendication 6,
dans lequel la couche de croissance comprend un catalyseur pour la croissance des nanofils (331, 332, 631, 632a, 632b, 732).

8. Dispositif d'analyse de biomolécules comprenant ledit dispositif de collecte de biomolécules selon l'une quelconque des revendications 1 à 7.

9. Procédé de collecte de biomolécules, comprenant :
la fourniture du dispositif de collecte de biomolécules selon les revendications 1 à 7 ; et
l'introduction d'une solution contenant une biomolécule dans le dispositif de collecte de biomolécules.

10. Procédé de collecte de biomolécules selon la revendication 9,
dans lequel ladite introduction de la solution comprenant la biomolécule dans le dispositif de collecte de biomolécules consiste à introduire en continu la solution comprenant la biomolécule.

11. Procédé selon la revendication 9 ou 10, comprenant en outre l'introduction d'un agent de dissociation dans le dispositif de collecte de biomolécules afin de dissocier la biomolécule capturée du nanofil.

12. Procédé selon l'une quelconque des revendications 9 à 11,
dans lequel la biomolécule comprend un microARN.

13. Procédé selon la revendication 12,
dans lequel la solution est de l'urine ou de la salive.

14. Méthode d'analyse d'expression d'ARN, comprenant :
la fourniture du dispositif d'analyse de biomolécules selon la revendication 8 et l'utilisation du dispositif de collecte de biomolécules pour collecter l'ARN ;
la mesure de l'ARN recueilli à l'aide du dispositif d'analyse de biomolécules ; et
l'estimation de la quantité d'expression de l'ARN dans le fluide corporel sur la base des données d'ARN mesurées.

15. Méthode selon la revendication 14,
dans lequel ladite estimation de la quantité d'expression de l'ARN dans le fluide corporel comprend la détermination d'un profil d'expression de l'ARN dans le fluide corporel.

16. Méthode selon la revendication 14 ou 15, dans lequel le fluide corporel est de l'urine ou de la salive.
